# EUROPÄISCHE PATENTSCHRIFT

(11) **EP 2 334 806 B1**
(45) Veröffentlichungstag und Bekanntmachung des Hinweises auf die Patenterteilung: **19.12.2012**
(21) Anmeldenummer: 09752720.4
(22) Anmeldetag: 07.10.2009
(51) Int. Cl.: C12Q 1/68, C12N 15/63

(54) **VERFAHREN ZUR IDENTIFIZIERUNG GENAKTIVIERENDER WIRKSTOFFE**
METHOD FOR IDENTIFYING GENE-ACTIVATING ACTIVE SUBSTANCES
PROCÉDÉ D'IDENTIFICATION DE PRINCIPES ACTIFS ACTIVATEURS GÉNIQUES

(30) Priorität: 07.10.2008 DE 102008050702
(43) Veröffentlichungstag der Anmeldung: 22.06.2011
(73) Patentinhaber: Thiesen, H.-J., 18055 Rostock (DE)
(72) Erfinder: Thiesen, H.-J., 18055 Rostock (DE)
(74) Vertreter: Schneider, Michael
(86) Internationale Anmeldenummer: PCT/EP2009/007195
(87) Internationale Veröffentlichungsnummer: WO 2010/040515

(56) Entgegenhaltungen:
- WO-A1-00/73434
- WO-A1-01/00815
- WO-A1-01/11037
- WO-A1-01/98507
- WO-A2-02/50259
- WO-A2-98/13502
- WO-A2-99/13077
- WO-A2-03/054148
- DEUSCHLE U ET AL: "TETRACYCLINE-REVERSIBLE SILENCING OF EUKARYOTIC PROMOTERS" MOLECULAR AND CELLULAR BIOLOGY, AMERICAN SOCIETY FOR MICROBIOLOGY, WASHINGTON, US, Bd. 15, Nr. 4, 1. April 1995 (1995-04-01), Seiten 1907-1914, XP000601654 ISSN: 0270-7306

## Beschreibung

Die Erfindung beruht auf einer Technologie zur Bestimmung von Leitstrukturen bzw. Wirkstoffen und Regulationswegen, die die Regulation von Transkriptionsprozessen bei menschlichen Krankheiten, wie Krebs modulieren.

Nachweissysteme, wie z.B. Luciferase-Genkonstrukte unter der Regulation von Promotor-Konstrukten, die cis-agierende Elemente enthalten, die wiederum als Bindungszellen für das Tetracyclin-Repressor-Protein dienen, müssen stabil in eukaryontische Zellen integriert werden. Ein zweites Konstrukt, welches den tet-Repressor im Verbund mit der TetKRAB-Domäne exprimiert, muss in dieselbe eukaryontische Zelle integriert werden. So eine Zelllinie wurde 1995 von Deuschle et al., Molecular and Cellular Biology, Vol. 15, April 1995, Seiten 1907 -1914, publiziert. Auf den Offenbarungsgehalt dieser Publikation wird hier ausdrücklich Bezug genommen bzw. deren Offenbarungsgehalt wird durch diese Bezugnahme inkorporiert. In Anwesenheit von Tetracyclin wird das Luciferasegen angeschaltet und in Abwesenheit von Tetracyclin abgeschaltet (Stand der Wissenschaft 1995).

WO 00/73434 A1 und WO 01/00815 A1 beschreiben Verfahren zur Identifikation von Wirkstoffen, welche die Repression der transkriptionellen Genexpression durch KRAB-Repressorproteine aufheben. WO 03/054148 A2 beschreibt ein Zn-Fingerbasierendes, Arzneimittel-kontrolliertes Genregulationssystem, bestehend aus einer KRAB-Repressordomäne, die an ein Zn-Finger-Monomer-TetR fusioniert ist. Durch Zugabe eines Liganden (Tetracyclin) wird die Bindung des dimeren Fusionsproteins an die Ziel-DNA unterbunden, so dass sich die Expression eines Reporterkonstruktes stromabwärts der Promotorsequenz ändert. WO 99/13077 A2 beschreibt ein Fusionsprotein aus dem tet Repressor (tetR) und der Krüppel-assoziierten Box (KRAB) des Kox1-Zn-Fingerproteins. Eine zweite Polynucleotidesequenz beinhaltet ein Reportergen, welches funktionell mit der Tetracyclin-Operatorsequenz verknüpft ist. Des Weiteren wird die induzierte Expression des Reportergens durch Zugabe von Tetracyclin oder Tetracyclin-Analoga beschrieben. WO 01/11037 A1 beschreibt ein System zur kontrollierten Transcription von Genen mit Tetracyclin als 'Inducer' und einem aus Kox1 und dem Tetracyclinerepressor (tetR) bestehenden Fusionsprotein. Durch Zugabe von Tetracyclin oder Tetracyclin-Analoga wird die durch KRAB-vermittelte Repression des Fusionsproteins aufgehoben.

Gemäß dieser Erfindung kann die Zelllinie benutzt werden, um Leitstrukturen zu ermitteln oder Wirkstoffe zu finden, die mit Transkriptionsprozessen in die eukaryontische Zelle interferieren.

Die Zelllinie TIS 10, 1995 in dem obigen Journal publiziert von Deuschle et al., kann benutzt werden, um Leitstrukturen in Komponentenbibliotheken zu identifizieren, die an der Modulation transkriptioneller Prozesse beteiligt sind.

Leitstrukturen, die ganz unterschiedliche Regulationswege adressieren, können identifiziert werden, falls diese Komponenten die Tet-KRAB vermittelte Repression an dem Luciferase-Zielkonstrukt aufheben.

Strukturell ganz unterschiedliche Komponenten können identifiziert werden.

KRAB-ZNF-Gene kodieren C₂H₂-Zinkfinger-Domänen zusammen mit der Krüppel-assoziierten Box (KRAB), initial identifiziert als Siebenerwiederholung von Leucinen in KOX1/ZNS10 (1; zum Überblick der humanen- und Mausgene, s. Datenbank Sys-ZNF, 2). Die KRAB-Domäne stellt eine der wirkungsvollsten Repressor-Domänen dar, die im Säugetiergenom gefunden wurden (3). Im Jahr 1995 : Die KRAB-Domäne fusioniert mit dem Tetracyclin-Repressor tetR beschrieb das erste Repressorsystem (tetR-KRAB), welches über ein kleines Molekül (Tetracyclin) reguliert werden konnte. Genetisch veränderte Hela-Zellen (TIS 10-Zellen) kodieren für ein stabil integriertes Luciferase-Reporter-Konstrukt zusammen mit tetR-Bindungsstellen sowie für ein TetR-KRAB-Expressionskonstrukt (4, 5). Mit dem Erscheinen der Tetrapoden erfolgte eine umfangreiche Expansion von ZNF-Genen (ZNF = Zinkfinger), die sich hauptsächlich während der Evolution der Säugetiergenome ereignete. Jedoch warten viele Eigenschaften dieser KRAB-ZNF-Gene noch darauf, entschlüsselt zu werden. TIS 10-Zellen stellen ein anwendungsfähiges System dar, um Substanzen (small molecules) zu identifizieren, die mit der KRAB-abhängigen Genexpression in Ab- oder Anwesenheit von Tetracyclin interferieren.

Mit der Unterstützung des Leibniz-Institutes für Pharmakologie konnte an 470 Komponenten der ChemBioNet Bibliothek im ersten Screen gezeigt werden, dass sie die Reporter-Gen-Expression beeinflussen, und sie wurden reanalysiert in Hela-Zellen, die transient mit unterschiedlichen Kontrollkonstrukten transfiziert waren. Die Komponenten mit den höchsten spezifischen Aktivitäten wurden strukturell mit allen 18.079 Komponenten, die in der ursprünglichen ChemBioNet Bibliothek und mit den Molekülen, die in der Pub Chem Bibliothek (etwa 19 Mio.) und in der DTP Bibliothek (237.771 Einträge) vorhanden sind, verglichen.

Am interessantesten ist: strukturell ähnliche so wie Tetracyclin-ähnliche Komponenten, die in der ChemBioNet Bibliothek vorkommen, zeigen keine vergleichbaren Luciferaseaktivitäten, darauf hinweisend, dass strukturell ähnliche Moleküle keine ähnlichen biologischen Funktionen ausüben. Schließlich, die effizientesten Komponenten, die mit der KRAB-vermittelten Repression interferieren, werden zur weiteren Analyse ausgewählt, um zu bestimmen, ob die Interferenz mit der KRAB-vermittelten Gen-Repression benutzt werden kann, um mögliche KRAB-ZNF gesteuerte Ziel-Gene mittels Affymetrix^{®}-basierter Mikro-Array-Expressionsanalyse zu identifizieren.

### Einführung

KRAB Zinkfingergene(-proteine)
- Große Familie mit mehr als 350 Genen im Menschen
- Zahlenmäßige Zunahme beginnend mit der Evolution der Tetrapoden
- Im allgemeinen ubiquitär mit relativ geringer Intensität exprimiert
- Gewöhnlich mehr als 5 Zinkfingerdomänen (oftmals mehr als 10) des C2H2-Typs
- Mutmaßlich DNA/RNA bindende Proteine
- KRAB-transkriptionelle Repressionsdomänen (Box A, B, b, B1, C)
- Funktionen größtenteils unbekannt

Eigenschaften der KRAB-vermittelten transkriptionellen Repression
- Bindung an die DNA in cis, d.h. Promoternähe, ist erforderlich
- Abschaltfunktion arbeitet effizient auch von weiter entfernten Positionen
- KRAB-vermittelte Repression dominiert die VP16 Aktivierungsdomäne
- Abschaltfunktion wird über den Intermediärfaktor (TIF1beta) übertragen
- Mechanismen der Repression beinhalten Chromatinmodifizierende Aktivitäten

### TIF1beta

- Mitglied der TRIM Familie
- RBCC Teil interagiert mit der KRAB Domäne
- Knock-outs sind embryonal letal in der Maus
- Interagiert mit Heterochromatin-HP1-Proteinen
- Komponenten der N-CoR1 und NuRD-Chromatin Remodelling Complexes
- Interagiert mit der H3-Histone-Methyltransferase SETDB1

Die Erfindung betrifft somit ein Verfahren zur Identifizierung von Wirkstoffen bzw. Leitstrukturen, welche die Repression der transkriptionellen Genexpression durch KRAB-Repressorproteine aufheben, unter Verwendung
a) mindestens eines Expressionskonstrukts, dass stromabwärts (also zum 3'-Ende der Nukleinsäuresequenz) von einer geeigneten Promoter-DNA-Sequenz mindestens eine DNA-Sequenz aufweist, die mindestens ein Fusionsprotein aus der KRAB-Repressordomäne vom menschlichen Kox1-Zinkfingerprotein und mindestens einem Tetracyclinrepressor (TetR) kodiert,
   und
b) mindestens eines Reporterkonstrukts, dass stromaufwärts von einer geeigneten Promoter-DNA-Sequenz mindestens einen Tetracyclinoperator, an den ein Tetracyclinrepressor binden kann, und stromabwärts von der Promoter-DNA-Sequenz mindestens eine DNA-Sequenz, die mindestens ein Reporterprotein kodiert, aufweist,
   bei dem
   das Expressionskonstrukt und das Reporterkonstrukt in einem geeigneten Expressionssystem mit dem potentiellen Wirkstoff inkubiert werden und aus der Expressionshöhe des Reporterproteins im Vergleich zu einer Kontrollprobe auf die Aufhebung einer durch die Kox1-KRAB-Domäne-vermittelten Repression geschlossen wird, und bei dem durch Zugabe von Tetracyclin als Co-Repressor die Bindung des TetR-KRAB-Fusionsproteins an den Tetracyclinoperator verhindert und die volle Expressionshöhe wieder hergestellt wird, was anzeigt, dass die Repression durch die KRAB-Domäne vermittelt wurde.

In dem Fusionsprotein des Expressionskonstrukts kann die KRAB-Repressordomäne z.B. vom menschlichen Kox1-Zinkfingerprotein stammen. Es kann aber eine beliebige KRAB-Repressordomäne verwendet werden.

Das Reporterprotein kann wegen der hohen Nachweisempfindlichkeit z.B. Luciferase oder z.B. das grünfluorezierende Protein sein.

Die Promoter-DNA-Sequenz kann z.B. ein CMV-Promoter (CMV = Cytomegalovirus) sein. Die Promoter-DNA-Sequenzen des Expressionskonstrukts und des Reporterkonstrukts können gleich oder unterschiedlich sein.

Das Expressionskonstrukt kann stromabwärts von der das Fusionsprotein kodierenden DNA-Sequenz ein Polyadenylierungssignal aufweisen, und das Reporterkonstrukt kann stromabwärts von der das Reporterprotein kodierenden DNA-Sequenz ein Polyadenylierungssignal aufweisen.

Das Expressionssystem für das Expressionskonstrukt und das Reporterkonstrukt kann jeweils ein Expressionsplasmid sein, dass Reporterprotein kodierenden DNA-Sequenz ein Polyadenylierungssignal aufweisen.

Das Expressionssystem für das Expressionskonstrukt und das Reporterkonstrukt kann jeweils ein Expressionsplasmid sein, dass beispielsweise in geeignete Zellen wie HeLa-Zellen eingeführt wird.

Wenn durch Zugabe von Tetracyclin als Co-Repressor die Bindung des TetR-KRAB-Fusionsproteins an den Tetracyclinoperator verhindert und die volle Expressionshöhe wieder hergestellt werden kann, zeigt dies an, dass die Repression durch die KRAB-Domäne vermittelt wurde.

### Figurenbeschreibung:

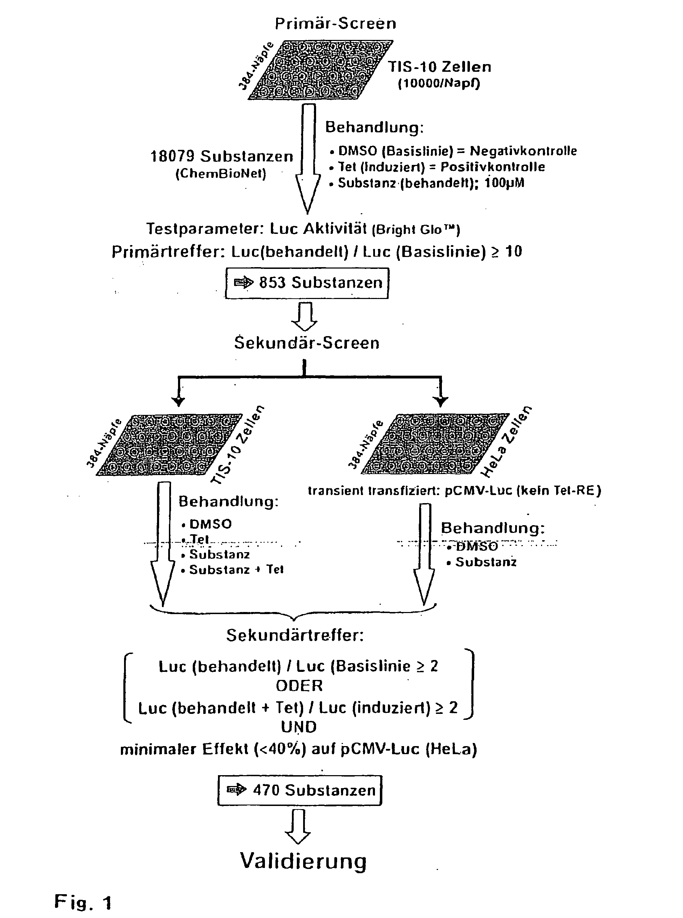

Fig. 1: Zeigt den experimentellen Ansatz des initialen Screenings, um chemische Verbindungen zu entdecken, welche das TetR-KRAB Repressionssystem in TIS-10 Zellen beeinflussen.
Fig. 2 : Zeigt die Luciferase-Aktivitäten von 14 Substanzen, ermittelt im 2. Screen unter unterschiedlichen Bedingungen verglichen mit der DMSO Kontrolle. Es sei auf die unterschiedlichen Gruppen hingewiesen.
   Gruppe I (Class I):
      Induktion von Luciferase (Luc) (Abwesenheit von Tet) UND Additive/synergist. Effekte (Anwesenheit von Tet)
   Gruppe II (Class II):
      Minimale Luciferase (Luc)-Induktion (Abwesenheit von Tet) UND Additive/synergist. Effekte (Anwesenheit von Tet)
   Gruppe III (Class III):
      Induktion von Luciferase (Luc) (Abwesenheit von Tet) ABER keine Additive/synergist. Effekte (Anwesenheit von Tet)

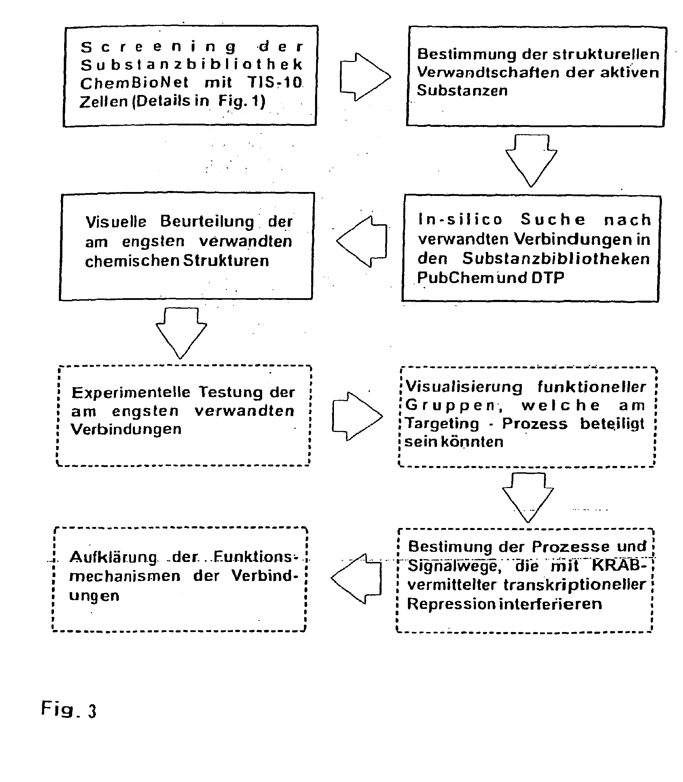

Fig. 3: Zeigt den Arbeitsablaufplan des Projektes zur Entdeckung und Aufklärung von kleinen chemischen Verbindungen, welche mit KRAB-vermittelter transkriptioneller Repression interferieren. Geschlossener Rahmen = schon realisierte Schritte; geschilderte Rahmen = Schritte in Planung

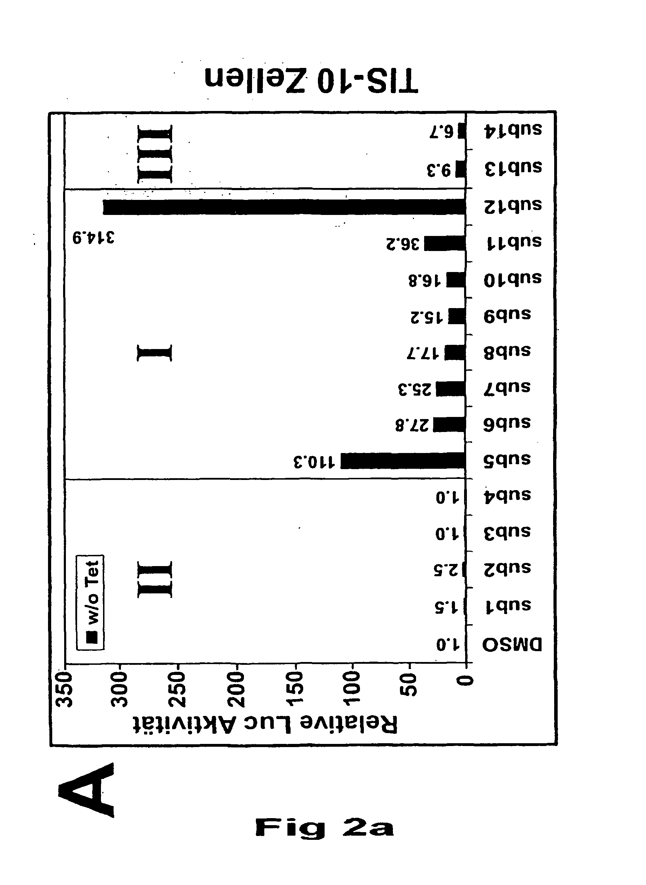

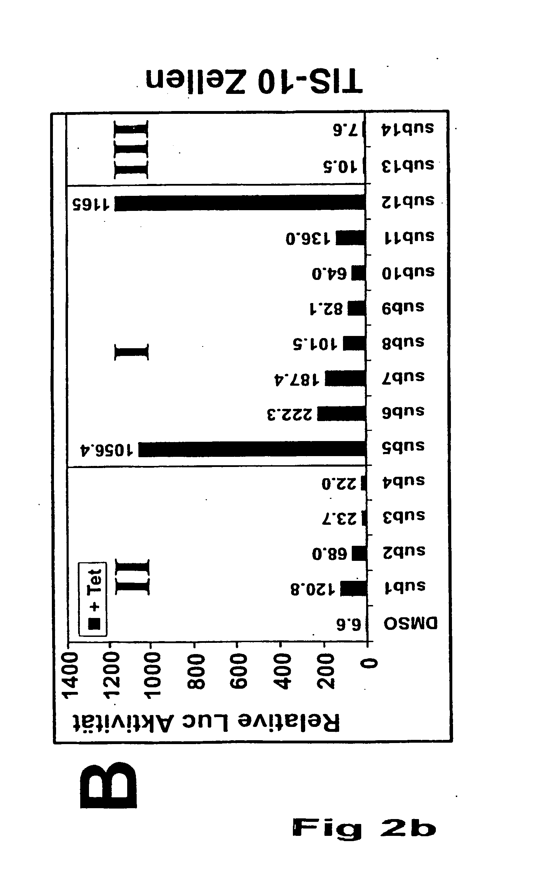

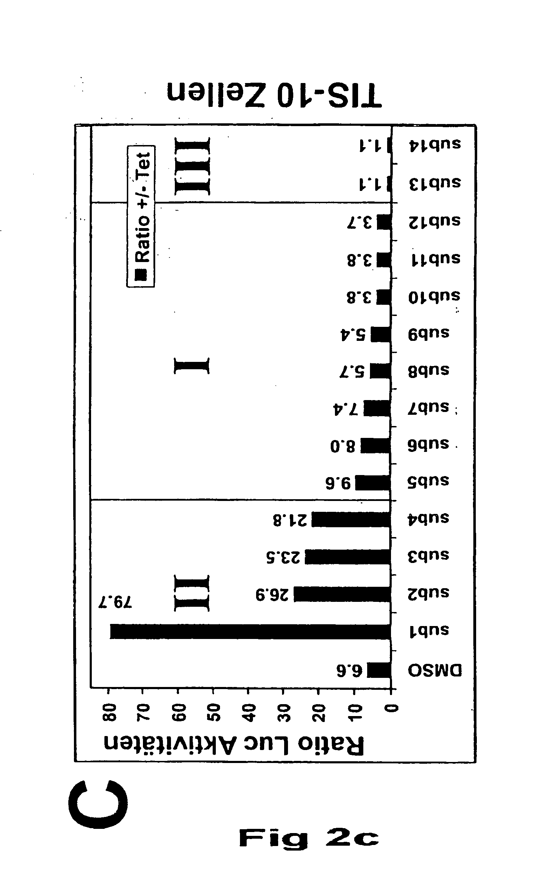

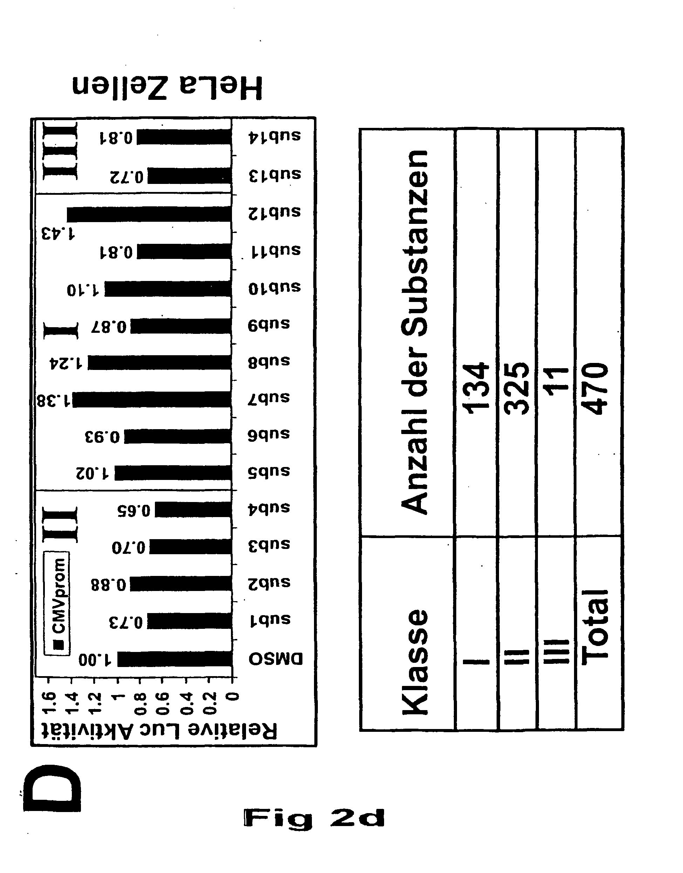

Fig. 4: Zeigt die auf der chemischen Struktur basierende hierarchische Gruppierung (Clustering) von 470 positiven Ergebnissen (hits) aus dem sekundären Screen stammend. Das Clustering basiert auf Strukturvergleichen, die mit Open Babel gemacht wurden, indem der Tanimoto-Koeffizient benutzt wurde. Ausgewählte Zweige des Baumes heben chemische Substanzen hervor, die eine mehr als >5, >10 oder > 70 fache Aktivierung der Luciferase in TIS-10 Zellen verglichen mit DMSO Kontrollen zeigen (siehe Beispiele in Fig. 2A; Pfeil = Position einiger Substanzen aus Fig. 2).
Fig. 5: Zeigt einen Auszug aus einem Baum mit den gleichen Daten wie in Fig. 4 mit der Ausnahme, dass die Struktur von Tetrazyklin (Tet, Pfeil) hinzugefügt wurde bevor das Clustering durchgeführt wurde. Nur die Hauptklade einschließlich Tetracyclin ist dargestellt.

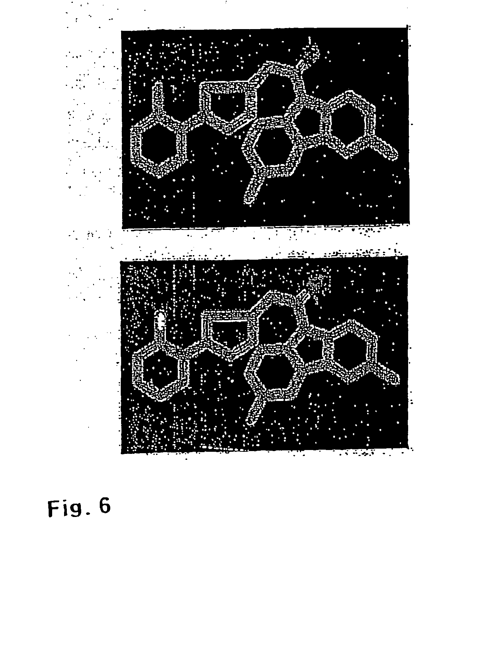

Fig. 6: Zeigt ein Beispiel für zwei aktive Komponenten, die direkte Nachbarn aufgrund des hierarchischem Clusterings in einem Unterbaum sind und strukturell sehr ähnlich sind. Die beiden Substanzen interagieren möglicherweise mit der gleichen Zielstruktur.

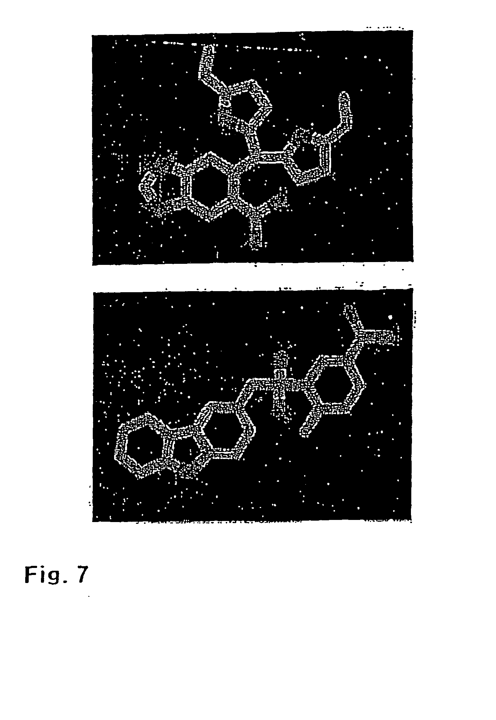

Fig. 7: Zeigt ein Beispiel für zwei aktive Komponenten, die direkte Nachbarn in einem Unterbaum aufgrund des hierarchischen Clusterings sind, aber strukturell sehr verschieden. Die beiden Komponenten interagieren möglicherweise mit unterschiedlichen Zielstrukturen.
Fig. 8: Zeigt In-silico-Vergleiche selektierter Substanzen mittels hierarchischem Clustering der Struktur (Open Babel) mit chemischen Substanzen in Datenbanken/ Bibliotheken ChemBioNet, DTP und PubChem.
   Beispiel für eine aktive Leitstruktur (markiert in rot, Pfeil). Es sei angemerkt, dass die nächsten Nachbarn (kurze Länge der Äste deuten auf nahe strukturelle Ähnlichkeiten hin) Kandidatenmoleküle für eine weiterführende experimentelle Austestung sind.
Fig. 9: Konkordanz Englisch / Deutsch Figurenlegende:
   KRAB Domäne: Siebenerwiederholung von Leucinen.
   Vergleich von Siebenerwiederholungen von Leucinen in ZNF10/KOX1 mit Leucin-Zipper-Strukturen in v-Fos, v-Jun, c-Myc, CEBP und GCN4 (Referenz 1)
   Lokalisation des ZNF10/Kox1-Proteins nach ektopischer Expression in HeLa Zellen..
   Modell der KRAB-Zinkfingerprotein-gesteuerten, TIF1beta vermittelten transkriptionellen Repression (Silencing).
Fig. 10: Zeigt das HeLa TIS-10 Zell-System:
   TIS10 Zellen enthalten z.B. zwei stabile Transgene: i) den Tet-Repressor fusioniert mit KOX1-KRAB (TetR-KRAB) als transkriptionelles Repressorprotein, dessen Bindung über das Hinzufügen von Tetracyclin (Tet) inhibiert werden kann und ii) ein Luciferase Reporter-Konstrukt, der einen starken CMV-Promoter und 7 aufwärts sitzende Tet-Operator-Stellen für die Bindung des TetR-Proteins enthält. In der Abwesenheit von Tet bindet TetR-KRAB an die TetO-Sequenzen und die Transkription des Luciferase-Reporters wird inhibiert, während in Anwesenheit von Tetracyclin (Tet) diese aufgehoben wird (Referenzen 4, 5).

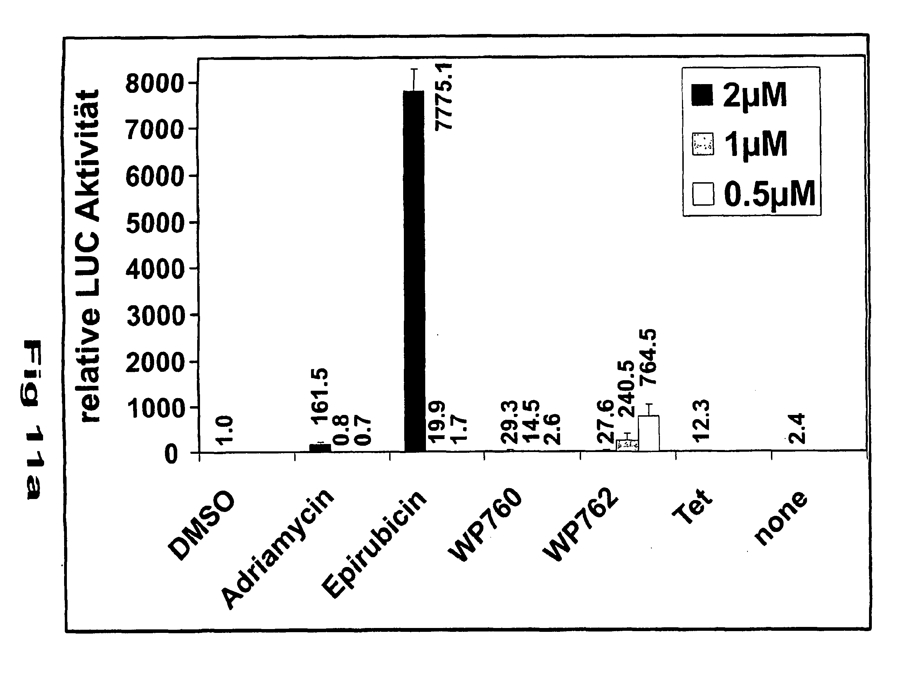

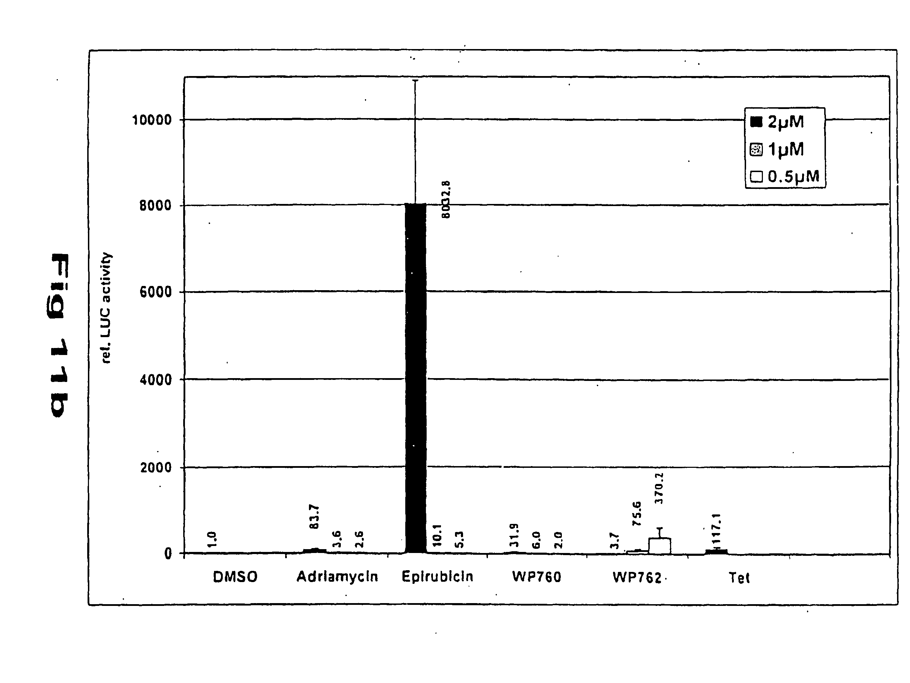

Figur 11: Luciferaseaktivität durch Aufhebung der KRAB-vermittelten Repression durch die Wirkstoffe Adriamycin, Epirubicin, WP760 und WP762. Es zeigt sich, dass die strukturell sehr ähnlichen Wirkstoffe Adriamycin und Epirubicin, die zur Klasse der Anthracycline gehören, mit dem erfindunggemäßen Verfahren hinsichtlich ihrer Wirkung differenziert werden können. Dies ergibt sich auch für die Wirkstoffe WP760 und WP762, die ebenfalls zu den Anthracycline gehören

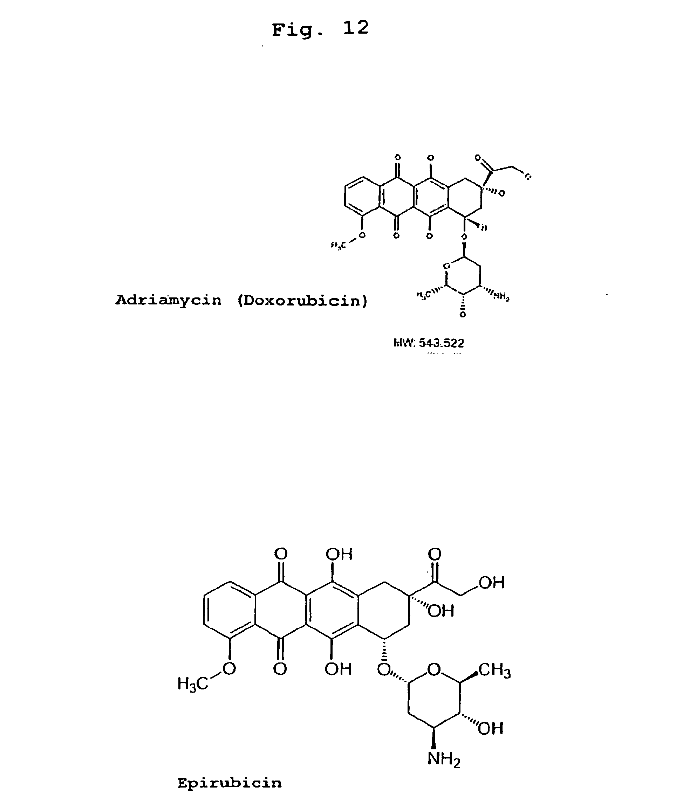

Fig. 12: Strukturformeln von Adriamycin (Doxorubicin) und Epirubicin

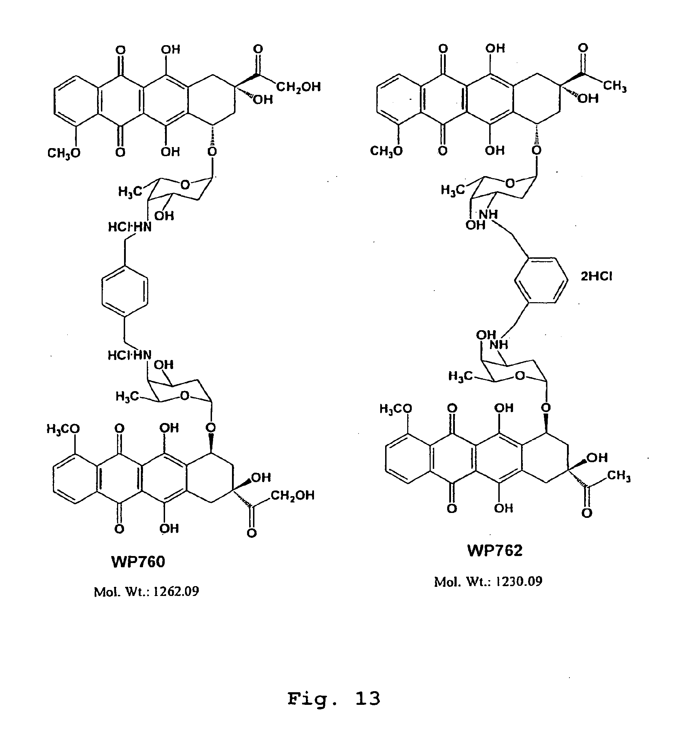

Fig. 13: Strukturformeln der Wirkstoffe WP760 und WP762

Anthracycline werden in der Klinik als Antitumormittel verwendet.

### Ergebnisse:

A. 470 Substanzen, die die KRAB-vermittelte Repression modifizieren, wurden in zwei Screening-Runden selektioniert.
B. Drei Klassen funktioneller Aktivitäten wurden definiert.
   - Gruppe I (Class I):: Luciferase-Reporter-Induktion UND additive/ synergistische Effekte mit Tetracyclin
   - GruppeII (Class I)I:: Luciferase-Reporter-Induktion nur in Anwesenheit von Tetracyclin
   - Gruppe III (Class III):: Luciferase-Reporter-Induktion aber keine addit ven/synergistischen Effekte mit Tetracyclin
C. Die Aktivitäten der Substanzen korrelieren nicht mit strukturellen Ähnlichkeiten
D. Aktive Substanzen werden in unterschiedlichen spezifischen Kladen gefunden, welches impliziert, dass zahlreiche Zielproteine und Regulationswege getroffen werden.
E. Die Aktivität ist nicht mit der strukturellen Ähnlichkeit zu Tetracyclin korreliert.

Viele Substanzen wurden entdeckt, die direkt oder indirekt mit der Repression eines stabil integrierten CMV-Promoter-Expressions-Konstrukts bedingt durch ein TetR-KRAB-Repressorprotein-Konstrukt interferieren.

### Referenzen:

1 Thiesen HJ. Multiple genes encoding zinc finger domains are expressed in human T cells. New Biol. 1990; 2:363-74.
2 SysZNF: the C2H2 Zinc Finger Gene database, submitted. Web: epgd.biosino.org/SysZNF/
3 Margolin JF, Friedman JR, Meyer WK, Vissing H, Thiesen HJ, Rauscher FJ 3rd. Krüppel-associated boxes are potent transcriptional repression domains. Proc Natl Acad Sci U S A. 1994; 91:4509-13.
4 Deuschle U, Meyer WK, Thiesen HJ. Tetracycline-reversible silencing of eukaryotic promoters. Mol Cell Biol. 1995;15:1907-14.
5 Lorenz P, Koczan D, Thiesen HJ. Transcriptional repression mediated by the KRAB domain of the human C2H2 zinc finger protein Kox1/ZNF10 does not require histone deacetylation. Biol Chem. 2001; 382:637-44.

## Patentansprüche

1. Verfahren zur Identifizierung von Wirkstoffen, welche die Repression der transkriptionellen Genexpression durch KRAB-Repressorproteine aufheben, unter Verwendung
a) mindestens eines Expressionskonstrukts, dass stromabwärts von einer geeigneten Promoter-DNA-Sequenz mindestens eine DNA-Sequenz aufweist, die mindestens ein Fusionsprotein aus der KRAB-Repressordomäne vom menschlichen Kox1-Zinkfingerprotein und mindestens einem Tetracyclik repressor (TYetR) kodiert, und
b) mindestens eines Reporterkonstrukts, dass stromaufwärts von einer geeigneten Promoter-DNA-Sequenz mindestens einen Tetracyclinoperator, an den ein Tetracyclinrepressor binden kann, und stromabwärts von der Promoter-DNA-Sequenz mindestens eine DNA-Sequenz, die mindestens ein Reporterprotein kodiert, aufweist,
bei dem
das Expressionskonstrukt und das Reporterkonstrukt in einem geeigneten Expressionssystem mit dem potentiellen Wirkstoff inkubiert werden und aus der Expressionshöhe des Reporterproteins im Vergleich zu einer Kontrollprobe auf die Aufhebung einer durch die Kox1 -KRAB-Dömane vermittelten Repression geschlossen wird,
und bei dem durch Zugabe von Tetracyclin als Co-Repressor die Bindung des TetR-KRAB-Fusionsproteins an den Tetracyclinoperator verhindert und die volle Expressionshöhe wieder hergestellt wird, was anzeigt, dass die Repression durch die KRAB-Domäne vermittelt wurde.

2. Verfahren nach einem der vorstehenden Ansprüche, wobei das Reporterprotein Luciferase ist.

3. Verfahren nach einem der vorstehenden Ansprüche, wobei die Promoter-DNA-Sequenz ein CMV-Promoter ist.

4. Verfahren nach einem der vorstehenden Ansprüche, wobei das Expressionskonstrukt stromabwärts von der das Fusionsprotein kodierenden DNA-Sequenz und das Reporterkonstrukt stromabwärts von der das Reporterprotein kodierenden DNA-Sequenz ein Polyadenylierungssignal aufweist.

5. Verfahren nach einem der vorstehenden Ansprüche, wobei das Expressionssystem für das Expressionskonstrukt und das Reporterkonstrukt jeweils ein Expressionsplasmid ist.

## Claims

1. A method for identifying active substances that stop the repression of transcriptional gene expression by KRAB repressor proteins, using
a) at least one expression construct that has downstream of an appropriate promoter DNA sequence at least one DNA sequence that encodes at least one fusion protein from the KRAB repressor domain of the human Kox1 zinc finger protein and at least one tetracycline repressor (TetR), and
b) at least one reporter construct that upstream of an appropriate promoter DNA sequence can bind at least one tetracycline operator to the one tetracycline repressor, and downstream of the promoter DNA sequence has at least one DNA sequence that encodes at least one reporter protein,
wherein
the expression construct and the reporter construct are incubated with the potential active substance in an appropriate expression system and from the expression level of the reporter protein in comparison with a control sample it is concluded that a repression mediated by the Kox1-KRAB domain has been stopped,
and wherein by adding tetracycline as a co-repressor the binding of the TetR-KRAB fusion protein to the tetracycline operator is prevented and the full expression level is reproduced, and this indicates that the repression has been mediated by the KRAB domain.

2. The method according to any of the preceding claims, the reporter protein being luciferase.

3. The method according to any of the preceding claims, the promoter DNA sequence being a CMV promoter.

4. The method according to any of the preceding claims, the expression construct downstream of the DNA sequence encoding the fusion protein and the reporter construct downstream of the DNA sequence encoding the reporter protein having a polyadenylation signal.

5. The method according to any of the preceding claims, the expression system for the expression construct and the reporter construct respectively being an expression plasmid.

## Revendications

1. Procédé d'identification de principes actifs qui suppriment la répression de l'expression génique transcriptionnelle par les protéines de répression KRAB, faisant appel à
a) au moins une construction d'expression, qui présente, en aval d'une séquence d'ADN promotrice appropriée, au moins une séquence d'ADN codant pour au moins une protéine de fusion composée d'un domaine de répression KRAB de la protéine en doigt de zinc Kox1 humaine et d'au moins un répresseur tétracycline (TetR),
et
b) au moins une construction rapporteuse, qui présente, en amont d'une séquence d'ADN promotrice appropriée, au moins un opérateur tétracycline auquel peut se lier un répresseur tétracycline, et en aval de la séquence d'ADN promotrice, au moins une séquence d'ADN codant pour au moins une protéine rapporteuse,
procédé dans lequel
la construction d'expression et la construction rapporteuse sont incubées avec le principe actif potentiel dans un système d'expression approprié et on conclut à la suppression d'une répression médiée par le domaine KRAB de Kox1 de par le niveau d'expression de la protéine rapporteuse par comparaison à un échantillon témoin,
et dans lequel l'ajout de tétracycline comme corépresseur empêche la liaison de la protéine de fusion TetR-KRAB à l'opérateur tétracycline et le plein niveau d'expression est rétabli, ce qui indique que la répression était médiée par le domaine KRAB.

2. Procédé selon la revendication 1, dans lequel la protéine rapporteuse est la luciférase.

3. Procédé selon l'une des revendications précédentes, dans lequel la séquence d'ADN promotrice est un promoteur du CMV.

4. Procédé selon l'une des revendications précédentes, dans lequel la construction d'expression en aval de la séquence d'ADN codant pour la protéine de fusion et la construction rapporteuse en aval de la séquence d'ADN codant pour la protéine rapporteuse d'ADN présentent un signal de polyadénylation.

5. Procédé selon l'une des revendications précédentes, dans lequel le système d'expression destiné à la construction d'expression et celui destiné à la construction rapporteuse sont respectivement un plasmide d'expression.
